Europäisches Patentamt

European Patent Office

Office européen des brevets

(11) Veröffentlichungsnummer: **0 446 730 B1**

(12)
# EUROPÄISCHE PATENTSCHRIFT

(45) Veröffentlichungstag der Patentschrift: **13.09.95**

(51) Int. Cl.6: **C12P 21/08**, G01N 33/577, B01D 39/00

(21) Anmeldenummer: **91103138.3**

(22) Anmeldetag: **02.03.91**

(54) **Monoklonale Antikörper gegen komplexierte und nicht komplexierte Komplexbildner zur Entfernung von Schwermetallen aus wässrigen Lösungen und zur Analytik.**

(30) Priorität: **07.03.90 DE 4007079**

(43) Veröffentlichungstag der Anmeldung:
**18.09.91 Patentblatt 91/38**

(45) Bekanntmachung des Hinweises auf die Patenterteilung:
**13.09.95 Patentblatt 95/37**

(84) Benannte Vertragsstaaten:
**AT BE CH DE DK ES FR GB GR IT LI LU NL SE**

(56) Entgegenhaltungen:
**WO-A-89/11298**

(73) Patentinhaber: **BEHRINGWERKE Aktiengesell-schaft**
**Postfach 1140**
**D-35001 Marburg (DE)**

(72) Erfinder: **Bosslet, Klaus, Dr.**
**Am Schlag 5**
**W-3550 Marburg (DE)**
Erfinder: **Hermentin, Peter, Dr.**
**Salzköppel 9**
**W-3550 Marburg (DE)**
Erfinder: **Seemann, Gerhard, Dr.**
**Auf der Ebert 1**
**W-3550 Marburg (DE)**

EP 0 446 730 B1

**Beschreibung**

Die Erfindung betrifft monoklonale Antikörper (MAk), die mit hoher Spezifität und Avidität an wasserlösliche Komplexone wie Ethylendiamintetraacetat (EDTA) oder Diethylentriaminpentaacetat (DTPA) binden und nach Komplexierung von EDTA oder DTPA mit Metallionen ihre hohe Avidität und Spezifität zu diesen Komplexonen behalten. Diese MAk können daher z.B. gekoppelt auf Filter oder sonstige Träger zur Entfernung von toxischen Schwermetallen benutzt werden, die mit EDTA oder DTPA komplexiert sind. Desweiteren sind diese MAk als Komponenten von Immunoassays (RIA, ELISA etc.) zum quantitativen Nachweis von EDTA bzw. DTPA in wässrigen Lösungen geeignet.

Schwermetalle, die mit Komplexonen wie EDTA verbunden sind, stellen im Bereich der Trinkwasserversorgung eine ernsthafte Gefährdung dar. Demzufolge ist der schnelle, exakte und quantitative Nachweis der Konzentration von EDTA von ebenso großer Bedeutung wie die Entfernung von EDTA-Metallkomplexen aus Abwässern oder dem Trinkwasser.

Bei unseren Arbeiten zur Gewinnung von MAk gegen hydrophile Komplexone im Rahmen der Tumortherapie ist es uns überraschenderweise gelungen, MAk zu erzeugen, die eine hohe Avidität sowohl gegen nicht komplexiertes als auch mit Metallen komplexiertes EDTA oder DTPA besitzen.

Die MAk wurden wie folgt hergestellt. Wie in den Beispielen einzeln ausgeführt wurde als Hapten Isothiocyanato-benzyl-DTPA auf humanes Serum-Albumin gekoppelt und mit Yttrium-Ionen komplexiert. Mit diesem Hapten-"Carrier"-Komplex wurden anschließend geeignete Mäuse immunisiert und die Milzen der Mäuse mit den höchsten anti-DTPA Antikörpertitern mit einer geeigneten Myelomzellinie fusioniert. Die entstandenen Hybridome wurden in dem nachfolgend beschriebenen spezifischen "Enzyme-linked Immunoassay (ELISA)" getestet.

Der ELISA bestand aus einer festen Phase, die mit einer human Serumalbumin (HSA)-Benzyl-DTPA enthaltenden Lösung beladen wurde. Der zu testende, den MAk enthaltende Überstand wurde mit freiem Komplexon bzw. dessen Metallionkomplexen vorinkubiert und seine Bindung an die spezifische feste Phase gemessen. Hierzu wurde ein Enzymamplifikationssystem benutzt, welches an einen Anti Maus-Immunglobulinantikörper gekoppelt ist. Die Einzelheiten dieser Methodik sind in Beispiel 2 und 3 beschrieben.

Mittels dieses Testsystems wurden MAk erhalten, welche die in Tabelle I beschriebenen Eigenschaften besitzen.

Diese MAk binden im Gegensatz zu vielen anderen anti-DTPA/EDTA MAk nicht an menschliche Normalgewebe, wie mittels der APAAP-Technik (Cordell et al., J. Histochem. Cytochem. 32: 219, 1984) auf kryopraservierten Geweben ermittelt wurde. Ein in vivo Einsatz dieser MAk im Bereich der Diagnostik und Therapie ist somit möglich.

Als Kompetitoren wurden die Komplexone DTPA und EDTA in nicht komplexierter sowie in komplexierter Form (Beispiel 4) eingesetzt. Zusätzlich wurden als Inhibitoren die strukturverwandten Verbindungen trans-Aconitsäure und 1,2-Diaminoethan verwendet (siehe Tab. I). Die in Tab. I vorgestellten Kompetitorüberschüsse, die zu einer 50%igen Inhibition der MAk-Antigenbindung führen, zeigen, daß die aufgeführten MAk eine starke Bindung sowohl an nicht komplexiertes EDTA bzw. DTPA als auch an mit Metallionen komplexiertes EDTA bzw. DTPA haben. Diese Charakteristika lassen eine Anwendung der MAk z.B. gekoppelt auf Filter oder andere feste Träger zur Entfernung von sowohl nicht komplexiertem EDTA oder DTPA sowie auch von EDTA- bzw. DTPA-Metallionkomplexen aus Flüssigkeiten zu. Ebenso sind Präzipitationsreaktionen von MAk-/EDTA-Metallionkomplexen zur Entfernung der toxischen Metallionen geeignet.

Besonders geeignet für diese Anwendung ist MAk BW 2050/535, dessen Interaktion mit EDTA und seinen Komplexen besonders stark ist.

Die Erfindung betrifft folglich hochaffine MAk gegen sowohl mit Metallionen komplexiertes als auch nicht komplexiertes EDTA bzw. DTPA, Verfahren zu ihrer Herstellung sowie ihre Verwendung als Bestandteil eines Diagnostikums oder Therapeutikums bzw. zur Entfernung toxischer Metallionen.

Die Erfindung ist ferner in den Beispielen weiter ausgeführt und in den Patentansprüchen enthalten.

**Beispiel 1:**

Herstellung von EDTA- bzw. DTPA-spezifischen MAk

Als Hapten wurde Isothiocyanato-benzyl-DTPA auf humanes Serumalbumin (HSA) als Carrier mit einem Derivatisierungsgrad von 19 Benzyl-DTPA Molekülen pro HSA-Molekül entsprechend der von N.W. Brechbiel et al., Inorganic Chemistry 25: 2772-2781 (1986) beschriebenen Methodik kovalent gekoppelt. 20 $\mu$g dieses Hapten-Carrier-Komplexes, in welchen unmarkiertes $YCl_3$ komplexiert wurde, wurden s.c. an Tag 0 mit Freundschem Adjuvans, an Tag 7 und 14 mit inkomplettem Freundschem Adjuvans und an Tag 21 mit

2

PBS in BALB/c Mäuse injiziert. An Tag 24 wurden die Milzen der Mäuse mit den höchsten anti-DTPA Antikörpertitern mit der SP210-Agl4 Myelomzellinie (Shulman et al., Nature 276: 269 (1976)) fusioniert. Entstehende Hybridome wurden in einem spezifischen ELISA (Beispiel 2) auf Produktion von hochaffinen MAk überprüft.

**Beispiel 2:**

Quantitativer Inhibitions-ELISA für MAk durch DTPA bzw. EDTA Komplexe

Als Trägermaterial wurden teilbare 96-well Polystyrol Mikrotiterplatten (U-Form) Typ B, Fa. Nunc, Nr. 4-60445 eingesetzt. Der Test wurde nach dem folgenden Protokoll durchgeführt.

(1) Pro Vertiefung werden 50 $\mu$l Y Benzyl-DTPA-HSA 19-Konjugat mit einer Konz. von 1 $\mu$g Konjugat pro ml PBS, pH 7.2, pipettiert und über Nacht bei Raumtemperatur (RT) inkubiert.

(2) Der Überstand wird abgesaugt und es wird 3 x mit 0,05 M Tris-Citrat- Puffer, pH 7.4, (Waschlsg. 1) gewaschen; (1x waschen $\triangleq$ 250 $\mu$l Waschlösung pro Vertiefung einfüllen, 2 min stehen lassen, absaugen).

(3) Wenn die Mikrotiterplatte nicht direkt benötigt wird, läßt man sie über Nacht auf Zellstoff bei RT stehen (Öffnung nach unten). Danach wird die Platte in Folien mit Trockenpatronen eingeschweißt (Fa. Gaplast, Postfach 529, 8100 Garmisch-Partenkirchen). Unter diesen Bedingungen sind die Platten bei +4° C mindestens 8 Wochen haltbar.

(4) 250 $\mu$l Blocklösung werden pro Vertiefung aufgetragen und 30 min bei 37° C inkubiert.

(5) Während des Blockierens erfolgt die Vorinkubation des verdünnten Hybridomüberstandes mit dem Kompetitor (siehe Beispiel 3 bzw. Beispiel 6).

(6) Von den zu testenden, entsprechend vorverdünnten und vorinkubierten Hybridomaüberständen werden pro Vertiefung 50 $\mu$l aufgetragen und 30 min bei Raumtemperatur inkubiert.

(7) Anschließend wird 3x mit Waschlösung 2 gewaschen.

(8) Anschließend werden 50 $\mu$l 1:500 in Blocklösung verdünnter, mit alkalischer Phosphatase markierter Ziege anti Maus IgG$_1$-Antikörper pro Vertiefung aufgetragen und 30 min bei Raumtemperatur inkubiert.

(9) Danach wird 3x mit Waschlösung für Enzygnost[R] (Behringwerke AG) gewaschen.

(10) Anschließend werden 50 $\mu$l 0,1 mM NADP zugegeben.

(11) Danach wird 30 min bei Raumtemperatur inkubiert.

(12) Während der Inkubation mit NADP wird das Verstärkersystem wie folgt angesetzt: pro Platte werden zu 2 Teilen p-Iodo-Nitrotetrazolium Violett (INT) und 1 Teil PBS, pH 7.2, zugegeben, dann 1 Teil Diaphorase, des weiteren wird 1 Teil ADH zupipettiert.

(13) 50 $\mu$l dieses Verstärkersystems werden pro Vertiefung zugegeben.

(14) Bei deutlichem Farbumschlag von durchsichtig nach rot wird die Reaktion mit 100 $\mu$l einer 0,1 N H$_2$SO$_4$-Lösung pro Vertiefung abgestoppt.

(15) Die Extinktionen werden bei 492 nm im TITERTEK[R] MULTISCAN vermessen. Als Blankwert werden 50 $\mu$l NADP mit 50 $\mu$l Verstärkerlösung und 100 $\mu$l 0,1 N H$_2$SO$_4$ eingesetzt.

Folgende Reagenzien wurden eingesetzt:

NADP           - Fa. Sigma Best.Nr. N-0505

INT               - Fa. Sigma Best.Nr. I-8377

ADH            - Fa. Sigma Best.Nr. A-3263

DIAPHORASE    - Fa. Sigma Best.Nr. D-2381

Waschlösung 2    - Fa. Behring, Best.Nr. 0SEW96, enthält Tween/PBS

Blocklösung: PBS, pH 7.2, wird durch Zugabe von Casein und 30minütiges Einrühren 3%ig an Casein gemacht und auf pH 7.4 eingestellt. Danach werden Partikel 10 min bei 4000 U/min abzentrifugiert.

Verdünnter mit alkalischer Phosphatase markierter Ziege anti Maus IgG$_1$-Antikörper (Fa. Southern Biotechnology Associates, Cat.Nr. 1080-04)

Herstellung von 0.1 mM NADP:

7.65 mg NADP lösen in 100 ml 20 mM Tris, 0.1 mM MgSO$_4$, pH 9.5; die Lösung kann bei -20° C mehrere Monate gelagert werden.

Herstellung von INT (p-Iodo-Nitrotetrazolium Violett):

2.5 mg/ml 30%igem Ethanol im Ultraschallbad lösen; immer frisch ansetzen.

Herstellung von Diaphorase:

1 mg Diaphorase/ml PBS, pH 7.2, wird portioniert bei -20° C gelagert.

Herstellung von Alkoholdehydrogenase:

0.5 mg ADH/ml PBS, pH 7.2, werden portioniert bei -20° C gelagert.

**Beispiel 3:**

Vorinkubation des Hybridomüberstandes mit dem Kompetitor

Die Maus-IgG-Konzentration in Hybridomüberständen kann mittels kommerziell erhältlicher quantitativer ELISA-Systeme bestimmt werden und ist Stand der Technik.

Anhand der Konzentrationsbestimmung im ELISA werden die Hybridomüberstände auf 1.25 $\mu$g/ml in PBS ohne $Ca^{++}$ und $Mg^{++}$ verdünnt.

Umrechnung von Gramm in Mol: 150 000 g = 1 Mol MAk

$$1.25 \ \mu g = 8.33 \times 10^{-12} \ Mol$$

Um ein Verhältnis 1 + 1 von MAk und Inhibitor zu haben, wurden zu 50 $\mu$l Hybridomüberstand mit einer Konzentration von 8.33 x $10^{-12}$ Mol/ml 10 $\mu$l Inhibitor mit einer um den Faktor 5 erhöhten Konzentration von 8.33 x $10^{-12}$ Mol/200 $\mu$l gegeben.

Der Hybridomüberstand wird mit 100 000fachem, 50 000-fachem, 10 000fachem, 5 000 fachem, 1 000 fachem und 100fachem Kompetitorüberschuß für 30 min. bei Raumtemperatur inkubiert. Hiervon werden 50 $\mu$l in den ELISA (siehe Beispiel 2, Punkt 6) pipettiert.

**Beispiel 4:**

Erzeugung der DTPA- bzw. EDTA-Komplexe

Die Komplex-Konstante von DTPA bzw. EDTA zu den in Tabelle I dargestellten Metallionen ist extrem hoch, so daß bei äquimolarer Mischung von DTPA bzw. EDTA mit diesen Metallionen eine komplette Sättigung zu erwarten ist. Die entsprechenden Metallionen wurden deswegen in einem 3fachen molaren Überschuß mit den DTPA bzw. EDTA inkubiert. Als Beispiel wurden 170 $\mu$l einer 10 mM Cadmiumsulfatlösung in aqua bidest (siehe Beispiel 5) mit 30 $\mu$l einer 0.028 molaren DTPA-Stammlösung in aqua bidest für 5 min bei Raumtemperatur inkubiert. Zumischung von 10 $\mu$l dieser Kompetitorlösung zu dem Hybridomüberstand führt zu einem 100 000fachen Überschuß an Kompetitor über den im Hybridomüberstand enthaltenen MAk. Niedrigere Verhältnisse von Kompetitor zu MAk wurden dadurch erzielt, daß die Kompetitorlösung entsprechend dem gewünschten molaren Überschuß (siehe Beispiel 3) in der jeweiligen Salzionenlösung verdünnt wurde.

**Beispiel 5:**

Quelle und relevante physicochemische Parameter der eingesetzen Metallionen

Von folgenden Metallionen wurden 10 mmolare Lösungen in aqua bidest hergestellt:

| | |
|---|---|
| Manganchlorid MG 161,88 Fa. Merck Nr. 5934 | Ionenradius Mn: 80 pm |
| Cadmiumsulfat MG 256,5 Fa. Riedel de Haen Nr. 31145 | Ionenradius Cd: 97 pm |
| Zinkchlorid MG 136,28 Fa. Merck Nr. 8816 | Ionenradius Zn: 74 pm |
| Kupfersulfat MG 159,61 Fa. Riedel de Haen Nr. 31294 | Ionenradius Cu: 96 pm |
| Yttriumchlorid MG 303,36 Fa. Aldrich Nr. 20,491-9 | Ionenradius Y: 92 pm |
| Blei(II)-nitrat MG 331,20 Fa. Riedel de Haen Nr. 31137 | Ionenradius Pb: 120 pm |

**Beispiel 6:**

Kompetitiver Enzymimmunoassay zur Bestimmung der EDTA- bzw. DTPA-Konzentration in wässrigen Lösungsmitteln

In dem in Beispiel 2 beschriebenen quantitativen Inhibitions-ELISA wird anstatt definierter Kompetitormengen Zeile 15-17, Schritt 5) eine wässrige Probe (50 $\mu$l) mit zu bestimmendem, unbekanntem EDTA-Gehalt hinzugegeben. Der weitere Test erfolgt wie in Beispiel 2, Schritt 6 - 15 beschrieben. Die Reduktion der Extinktion ist proportional der Konzentration an EDTA bzw. DTPA in der unbekannten Probe. Nach

4

Aufstellung einer Eichgeraden durch Zugabe definierter EDTA- bzw. DTPA- Konzentrationen und entsprechender Vorverdünnung der zu testenden unbekannten Probe läßt sich die EDTA- bzw. DTPA-Konzentration in der unbekannten Probe quantitativ bestimmen.

**Beispiel 7:**

Beschreibung des besonders geeigneten MAk BW 2050/535

Das $V_H$ und $V_L$ Gen des MAk BW 2050/535 wurde nach der von Orlandi et al. (Proc. Natl. Acad. Sci. U.S.A. 86, 3833 (1989)) beschriebenen Methode kloniert und anschließend sequenziert.

Die Nukleinsäuresequenzen des $V_H$ Gens des MAk BW 2050/535 ist in Tab. II und die des $V_L$ Gens ist in Tab. III dargestellt. Die Sequenz des $V_H$ Gens umfaßt die kodierende Sequenz für die Aminosäuren 4 bis 110 des reifen Proteins.

Die Sequenz des $V_L$ Gens umfaßt die kodierende Sequenz für die Aminosäuren 3 bis 106 des reifen Proteins.

Tabelle I

Quantitativer Inhibitionstest von MAk durch DTPA bzw. EDTA Komplexe

molarer Überschuß an Kompetitor, der zu 50 % Inhibition der Bindung an das Festphasenantigen führt.

| MAk-Nr. | DTPA-Y | DTPA | DTPA-Mn | DTPA-Cd | DTPA-Zn | DTPA-Cu | DTPA-Pb | 1,2-Diami-noethan |
|---|---|---|---|---|---|---|---|---|
| 2050/174 | $10^4$ | $10^3$ | $10^2$ | $10^2$ | $5 \times 10^3$ | $5 \times 10^3$ | $10^3$ | keine Inhibition bis $10^5$ |
| 2050/531 | $5 \times 10^4$ | $10^3$ | $10^2$ | $10^2$ | $5 \times 10^3$ | $5 \times 10^3$ | $5 \times 10^3$ | " |
| 2050/532 | $5 \times 10^4$ | $10^3$ | $10^2$ | $10^2$ | $5 \times 10^3$ | $5 \times 10^3$ | $5 \times 10^3$ | " |
| 2050/534 | $5 \times 10^4$ | $10^3$ | $10^2$ | $10^2$ | $5 \times 10^3$ | $5 \times 10^3$ | $5 \times 10^3$ | " |
| 2050/535 | $10^4$ | $10^2$ | $10^2$ | $10^2$ | $10^3$ | $10^3$ | $10^3$ | " |

Tabelle I - Fortsetzung

| MAk-Nr. | trans-Aconitsäure | EDTA-Y | EDTA | EDTA-Mn | EDTA-Cd | EDTA-Zn | EDTA-Cu | EDTA-Pb |
|---|---|---|---|---|---|---|---|---|
| 2050/174 | keine In-hibition bis $10^5$ | $10^2$ | $10^3$ | $10^3$ | $10^3$ | $10^3$ | $10^3$ | $5x10^3$ |
| 2050/531 | " | $10^3$ | $10^3$ | $10^3$ | $10^3$ | $10^3$ | $10^2$ | $10^5$ |
| 2050/532 | " | $10^2$ | $10^3$ | $10^3$ | $10^3$ | $10^2$ | $5x10^3$ | $10^5$ |
| 2050/534 | " | $10^2$ | $10^3$ | $10^2$ | $10^2$ | $10^3$ | $10^3$ | $5x10^3$ |
| 2050/535 | " | $10^2$ | $10^2$ | $10^2$ | $10^2$ | $10^2$ | $10^2$ | $10^2$ |

EP 0 446 730 B1

EP 0 446 730 B1

## Tabelle II:

```
1     /    1                                    31    /   11
ctg cag cag tct ggg gca gag ctt gtg aag cca ggg gcc tca gtc aag ttg tcc tgc aca
leu gln gln ser gly ala glu leu val lys pro gly ala ser val lys leu ser cys thr
61    /   21                                    91    /   31
gct tct ggc tta aac att aaa gac acc tat ata aac tgg gtg aag cag agg cct gaa cag
ala ser gly leu asn ile lys asp thr tyr ile asn trp val lys gln arg pro glu gln
121   /   41                                    151   /   51
ggc ctg gag tgg att gga agg att ggt cct gcg aat ggt aat act aaa tat gac ccg aag
gly leu glu trp ile gly arg ile gly pro ala asn gly asn thr lys tyr asp pro lys
181   /   61                                    211   /   71
ttc cag ggc aag gcc act tta aca gca gac aca tcc tcc aac aca gcc tac cta caa ctc
phe gln gly lys ala thr leu thr ala asp thr ser ser asn thr ala tyr leu gln leu
241   /   81                                    271   /   91
agc agc ctg aca tct gag gac act gcc gtc tat tac tgt tct aga aga tgg ttc ttt ctt
ser ser leu thr ser glu asp thr ala val tyr tyr cys ser arg arg trp phe phe leu
301   /  101
tac tgg ggc caa ggg acc acg gtc acc
tyr trp gly gln gly thr thr val thr
```

## Tabelle III:

```
  1 /                                       31 / 11
cag ctg act cag gaa tct gca ctc acc aca   cct gaa aca gtc aca ctc act tgt
gln leu thr gln glu ser ala leu thr thr   pro glu thr val thr leu thr cys

 61 /                                       91 / 31
cgc tca agt act ggg gct gtt aca act        tat aac agt tac gcc acc tgg gtc caa gaa aaa cca
arg ser ser thr gly ala val thr thr        tyr asn ser tyr ala thr trp val gln glu lys pro

121 /                                      151 / 51
gat cat tta ttc act ggt cta ata ggt        aag aac cga gct ccg ggt gtt cct gcc
asp his leu phe thr gly leu ile gly        lys asn arg ala pro gly val pro ala

181 /                                      211 / 71
aga tca ggc tcc ctg att gga gac aag        gct ctc acc atc aca ggg gca cag act
arg ser gly ser leu ile gly asp lys        ala leu thr ile thr gly ala gln thr

241 /                                      271 / 91
gag gag gca ata tat ttc tgt gct cta        tac agc aac cac tgg ttc ggt gga
glu glu ala ile tyr phe cys ala leu        tyr ser asn his trp phe gly gly

301 /
ggg acc aag ctg aag atc
gly thr lys leu lys ile
```

**Patentansprüche**

**Patentansprüche für folgende Vertragsstaaten : AT, BE, CH, DE, DK, FR, GB, GR, IT, LI, LU, NL, SE**

1. Hochaffine monoklonale Antikörper (MAk), die mit etwa gleicher Spezifität und Avidität gegen EDTA oder DTPA im komplexierten oder nichtkomplexierten Zustand reagieren.

2. Verfahren zur Herstellung von MAk nach Anspruch 1, dadurch gekennzeichnet, daß Isothiocyanato-benzyl-DTPA oder Isothiocyanatobenzyl EDTA gekoppelt an HSA zur Immunisierung eingesetzt wird.

3. Verfahren nach Anspruch 2, dadurch gekennzeichnet, daß hochaffine MAk durch einen Kompetitions-ELISA mit HSA-Benzyl-DTPA oder HSA-Benzyl-EDTA als Festphase isoliert werden.

4. Verwendung von MAk nach Anspruch 1 zur Reinigung von mit Schwermetallionen verunreinigten wässrigen Flüssigkeiten.

5. Filtermaterialien, die MAk nach Anspruch 1 enthalten.

6. Verwendung von MAk nach Anspruch 1 zur quantitativen Bestimmung der EDTA oder DTPA Konzentration in wässrigen Lösungen mittels Immunoassays.

7. Immunoassays, die MAk nach Anspruch 1 enthalten.

8. Verfahren zur quantitativen Bestimmung von EDTA oder DTPA in wässrigen Lösungen, dadurch gekennzeichnet, daß MAk nach Anspruch 1, vorzugsweise in Form eines geeigneten Immunoassays, eingesetzt werden.

9. MAk nach Anspruch 1, dadurch gekennzeichnet, daμ die $V_H$ und $V_L$ Gene, die in Tab. II und Tab. III dargestellten Nukleinsäuresequenzen besitzen.

**Patentansprüche für folgenden Vertragsstaat : ES**

1. Verfahren zur Herstellung hochaffiner monoklonaler Antikörper (MAk) die mit etwa gleicher Spezifität und Avidität gegen EDTA oder DTPA im komplexierten oder nicht komplexierten Zustand reagieren, dadurch gekennzeichnet, daß Isothiocyanato-benzyl-DTPA oder Isothiocyanato-benzyl-EDTA als Hapten gekoppelt an humanes Serumalbumin (HSA) als "Carrier" zur Immunisierung eingesetzt wird und die bezeichneten MAK aus den erzeugten Hybridomen isoliert werden.

2. Verfahren nach Anspruch 1, dadurch gekennzeichnet, daß die bezeichneten MAk aus den resultierenden Hybridomen durch einen Kompetitions-ELISA mit HSA-Benzyl-DTPA oder HSA-Benzyl-EDTA als Festphase isoliert werden.

3. Verfahren zur Reinigung von mit Schwermetallionen verunreinigten wässrigen Flüssigkeiten, dadurch gekennzeichnet, daß Filtermaterialien enthalten nach Anspruch 1 oder Anspruch 2 hergestellte MAk eingesetzt werden oder nach Anspruch 1 oder Anspruch 2 hergestellte MAk zugegeben und anschließend abgetrennt werden.

4. Verfahren zur quantitativen Bestimmung von EDTA oder DTPA in wässrigen Lösungen, dadurch gekennzeichnet, daß nach Anspruch 1 oder Anspruch 2 hergestellte MAk, vorzugsweise in Form eines geeigneten Immunoassays, eingesetzt werden.

**Claims**
**Claims for the following Contracting States : AT, BE, CH, DE, DK, FR, GB, GR, IT, LI, LU, NL, SE**

1. A monoclonal antibody (mAb) which has a high affinity and reacts with about the same specificity and avidity to EDTA or DTPA in the complexed or noncomplexed state.

2. A process for the preparation of an mAb as claimed in claim 1, which comprises using isothiocyanatobenzyl-DTPA or isothiocyanatobenzyl-EDTA coupled to HSA For the immunization.

3. The process as claimed in claim 2, wherein an mAb with a high affinity is isolated by means of a competitive ELISA with HSA-benzyl-DTPA or HSA-benzyl-EDTA as solid phase.

4. The use of an mAb as claimed in claim 1 for purifying aqueous liquids contaminated with heavy metal ions.

5. A filter material which contains mAb as claimed in claim 1.

6. The use of an mAb as claimed in claim 1 for the quantitative determination of the EDTA or DTPA concentration in aqueous solutions by means of immunoassays.

7. An immunoassay which comprises an mAb as claimed in claim 1.

8. A process for the quantitative determination of EDTA or DTPA in aqueous solutions, which comprises using an mAb as claimed in claim 1, preferably in the form of a suitable immunoassay.

9. An mAb as claimed in claim 1, wherein the $V_H$ and $V_L$ genes possess the nucleic acid sequences shown in Tab. II and Tab. III.

**Claims for the following Contracting State : ES**

1. A process for the preparation of a monoclonal antibody (mAb) which has a high affinity and reacts with about the same specificity and avidity to EDTA or DTPA in the complexed or noncomplexed state, which comprises using isothiocyanatobenzyl-DTPA or isothiocyanatobenzyl-EDTA as hapten coupled to human serum albumin (HSA) as "carrier" for the immunization, and isolating the mAb referred to from the hybridomas generated.

2. The process as claimed in claim 1, wherein the mAb referred to is isolated from the resulting hybridomas by means of a competitive ELISA with HSA-benzyl-DTPA or HSA-benzyl-EDTA as solid phase.

3. A process for purifying aqueous liquids contaminated with heavy metal ions, which comprises using filter materials containing an mAb prepared as claimed in claim 1 or claim 2, or adding and subsequently removing an mAb prepared as claimed in claim 1 or claim 2.

4. A process for the quantitative determination of EDTA or DTPA in aqueous solutions, which comprises using an mAb prepared as claimed in claim 1 or claim 2, preferably in the form of a suitable immunoassay.

**Revendications**

**Revendications pour les Etats contractants suivants : AT, BE, CH, DE, DK, FR, GB, GR, IT, LI, LU, NL, SE**

1. Anticorps monoclonaux (AcM) à haute affinité qui réagissent, à l'état complexé ou non complexé, avec approximativement la même spécificité et la même avidité contre l'EDTA ou le DTPA

2. Procédé pour la Production d'AcM selon la revendication 1, caractérisé en ce que, pour l'immunisation, on utilise de l'isothiocyanatobenzyl-DTPA ou du isothiocyanatobenzyl-EDTA couplés à de l'albumine de sérum humain (HSA).

3. Procédé selon la revendication 2, caractérisé en ce que les AcM à haute affinité sont isolés par un essai ELISA compétitif avec du HSA-benzyl- DTPA ou du HSA-benzyl-EDTA en tant que phase solide.

4. Utilisation d'AcM selon la revendication 1, pour l'épuration de liquides aqueux contaminés par des ions de métaux lourds.

5. Matériaux filtrants qui contiennent des AcM selon la revendication 1.

6. Utilisation d'AcM selon la revendication 1, pour la détermination quantitative, au moyen d'essais immunologiques, de la concentration d'EDTA ou de DTPA dans des solutions aqueuses.

7. Essais immunologiques qui contiennent des AcM selon la revendication 1.

8. Procédé pour la détermination quantitative d'EDTA ou de DTPA dans des solutions aqueuses, caractérisé en ce que l'on utilise des AcM selon la revendication 1, de préférence sous forme d'un essai immunologique approprié.

9. AcM selon la revendication 1, caractérisé en ce que les gènes $V_H$ et $V_L$ comportent les séquences d'acide nucléique représentées dans les tableaux II et III.

**Revendications pour l'Etat contractant suivant : ES**

1. Procédé pour la production d'anticorps monoclonaux (AcM) à haute affinité qui réagissent, à l'état complexé ou non complexé, avec approximativement la même spécificité et la même avidité contre l'EDTA ou le DTPA caractérisé en ce que, pour l'immunisation, on utilise de l'isothiocyanatobenzyl-DTPA ou de l' isothiocyanatobenzyl-EDTA, en tant qu'haptène, couplés à de l'albumine de sérum humain (HSA) en tant que "porteuse", et on isole à partir des hybridomes produits les AcM indiqués.

2. Procédé selon la revendication 1, caractérisé en ce que les AcM indiqués sont isolés, à partir des hybridomes résultants, par un essai ELISA compétitif avec du HSA-benzyl-DTPA ou du HSA-benzyl-EDTA en tant que phase solide.

3. Procédé pour l'épuration de liquides aqueux contaminés par des ions de métaux lourds, caractérisé en ce que l'on utilise des matériaux filtrants contenant des AcM produits selon la revendication 1 ou la revendication 2 ou on ajoute des anticorps produits selon la revendication 1 ou la revendication 2 et ensuite on les sépare.

4. Procédé pour la détermination quantitative d'EDTA ou de DTPA dans des solutions aqueuses, caractérisé en ce que l'on utilise des AcM produits selon la revendication 1, ou la revendication 2, de préférence sous forme d'un essai immunologique approprié.

12